Europäisches Patentamt

European Patent Office

Office européen des brevets

(11) Veröffentlichungsnummer: **0 250 904 B1**

# EUROPÄISCHE PATENTSCHRIFT

(45) Veröffentlichungstag der Patentschrift: **15.04.92**

(21) Anmeldenummer: **87108011.5**

(22) Anmeldetag: **03.06.87**

(51) Int. Cl.⁵: **A61L 2/26**

(54) **Packung mit einem darin enthaltenen, mit einem nicht sterilisierbaren Wirkstoff beladenen Wirkstoffträger und Verfahren zu deren Herstellung.**

(30) Priorität: **02.07.86 DE 3622237**

(43) Veröffentlichungstag der Anmeldung:
**07.01.88 Patentblatt 88/01**

(45) Bekanntmachung des Hinweises auf die
Patenterteilung:
**15.04.92 Patentblatt 92/16**

(84) Benannte Vertragsstaaten:
**AT BE CH DE ES FR GB GR IT LI LU NL SE**

(56) Entgegenhaltungen:
**WO-A-83/00430**
**DE-A- 2 952 733**
**FR-A- 2 543 110**

**REMINGTON'S PHARMACEUTICAL SCIENCES, Ausgabe 16, 1980, Seite 1483, Mack Publishing Co.; "Freeze-drying"**

(73) Patentinhaber: **Dr. Ruhland Nachf. GmbH**
**Stadtplatz 7**
**W-8425 Neustadt/Donau(DE)**

(72) Erfinder: **Die Erfinder haben auf ihre Nennung verzichtet**

(74) Vertreter: **Brauns, Hans-Adolf, Dr. rer. nat. et al**
**Hoffmann, Eitle & Partner, Patentanwälte**
**Arabellastrasse 4**
**W-8000 München 81(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 250 904 B1

## Beschreibung

Die Erfindung betrifft eine Packung mit einem darin enthaltenen, mit einem nicht sterilisierbaren Wirkstoff beladenen Wirkstoffträger. Sie betrifft auch ein Verfahren zur Herstellung einer solchen Verpackung.

Es ist bekannt, Arzneimittelträger mit Arzneimitteln zu beladen. Solche Arzneimittelträger können für eine lokale Behandlung verwendet werden. Durch die lokale Applikation werden im Körper lokale Arzneimittelkonzentrationen erreicht. Dadurch können die bei oraler bzw. intravasaler Anwendung verbundenen systemischen Nebenwirkungen weitgehend verhindert werden.

Die in der Regel im Rahmen chirurgischer Maßnahmen implantierbaren Materialien müssen den strengen Anforderungen hinsichtlich der Sterilität genügen. Häufig werden vom Chirurgen nach Ausräumung infektiöser Herde bzw. Entfernung von Tumoren bioresorbierbare Arzneimittelträger implantiert, die lokal eine antibiotische oder cytostatische Wirkung erreichen. Bei großflächigen Resektionen kann vor allem an parenchymatösen Organen der Einsatz von Materialien mit blutstillenden Eigenschaften in Kombination mit Arzneistoffen notwendig sein.

Bioresorbierbare Arzneimittelträger sind beispielsweise Materialien biologischen Ursprungs, wie Fibrin, Kollagen, oxidierte Cellulose, Chitin oder Derivate davon, sowie auch synthetische Polymere wie Polymilchsäure oder Polyglykolsäure. Diese Arzneistoffträger können in der Regel mittels gängiger Verfahren sterilisiert werden, z.B. durch Bestrahlung mit Gamma-Strahlen, oder durch eine Behandlung mit Ethylenoxid. Eine Reihe von Arzneistoffen, insbesondere aber Arzneistoffe mit Proteincharakter, lassen sich jedoch nicht mittels chemischer oder physikalischer Verfahren sterilisieren, weil sie unter den Sterilisationsbedingungen verändert werden, z.B. ein Abbau erfolgt. In diesem Fallist es erforderlich, daß schon die Herstellung des mit dem Arzneimittel beladenen Arzneistoffträgers unter aseptischen Bedingungen erfolgt, weil nachträglich eine Sterilisierung nicht mehr möglich ist.

Aus DE-A-2 952 733 ist eine sterilisationsfähige Verpackung aus einer thermoplastischen Kunststoffolie bekannt, die eine durchsichtige Deckelfolie und eine Faserfolie aufweist. Durch diese Faserfolie ist eine Gas-, Dampf- oder Strahlen-Sterilisation möglich. Weiterhin ist aus Remington's Pharmaceutical Sciences, 16. Ausgabe, 1980, Seite 1483 die Lyophilisierung von hitzelabilen Substanzen und das aseptische Auffüllen von steril-filtrieren Lösungen in sterilisierte Behälter mit anschließender Gefriertrocknung bekannt.

Es besteht jedoch die Notwendigkeit, eine Verpackung für einen mit einem nicht sterilisierbaren Wirkstoff beladenen Arzneimittelträger zu schaffen, in welcher der wirkstoffbeladene Arzneimittelträger in steriler Form vorliegt und als solcher vom Chirurgen nach Öffnung der Packung entnommen und direkt verwendet werden kann. Dabei muß sichergestellt werden, daß die beim Beladen des Wirkstoffträgers verwendeten Hilfsmittel, wie Lösungsmittel, einschließlich Wasser, aus der Verpackung entfernt werden.

Es müssen die folgenden Bedingungen erfüllt sein:
Die für die Behälter verwendeten Materialien müssen den mit dem Wirkstoff beladenen Arzneistoffträger ausreichend schützen. Glas und thermostabile Kunststoffe sowie Kunststoffe, die auch für eine Begasung mit Ethylenoxid geeignet sind, entsprechen diesen Anforderungen.

Die Behälter müssen so gebaut sein, daß eine aseptische homogene Beladung des Arzneimittelträgers mit dem Arzneimittel gewährleistet ist. Dies geschieht am besten durch ein in dem Behälter eingearbeitetes Septum.

Die Entfernung von Lösungsmitteln durch eine semipermeable und bakteriendichte Membran muß durch herkömmliche und einfache Methoden möglich sein, z.B. durch Gefriertrocknen oder Abziehen von nicht-wässrigen Lösungsmitteln im Vakuum. Dabei müssen die für die Verpackung verwendeten Materialien den bei der Gefriertrocknung auftretenden Belastungen ebenso gewachsen sein, wie einer Temperaturbelastung im Falle des Abziehens von nicht-wässrigen Lösungsmitteln bei höheren Temperaturen.

Der steril verpackte, wirkstoffbeladene Arzneimittelträger muß unter Einhaltung von sterilen Kautelen durch den Operateur entnehmbar sein.

Die der Erfindung zugrundeliegende Aufgabe, eine Packung mit einem darin enthaltenen, mit einem nicht sterilisierbaren Wirkstoff beladenen Wirkstoffträger zur Verfügung zu stellen, in welcher der Wirkstoffträger mit dem Wirkstoff in steriler Form vorliegt, wird erfindungsgemäß dadurch gelöst, daß man die folgenden Verfahrensschritte anwendet:

a) den Arzneimittelträger in eine Wanne aus Kunststoff einbringt,

b) die Wanne mit einer semipermeablen Membran verschließt,

c) den verschlossenen Behälter sterilisiert,

d) den Wirkstoff in gelöster oder suspendierter Form in einem Lösungsmittel durch ein Septum der Wanne oder der Membran einbringt, und

e) das Lösungsmittel durch die semipermeable Membran entfernt.

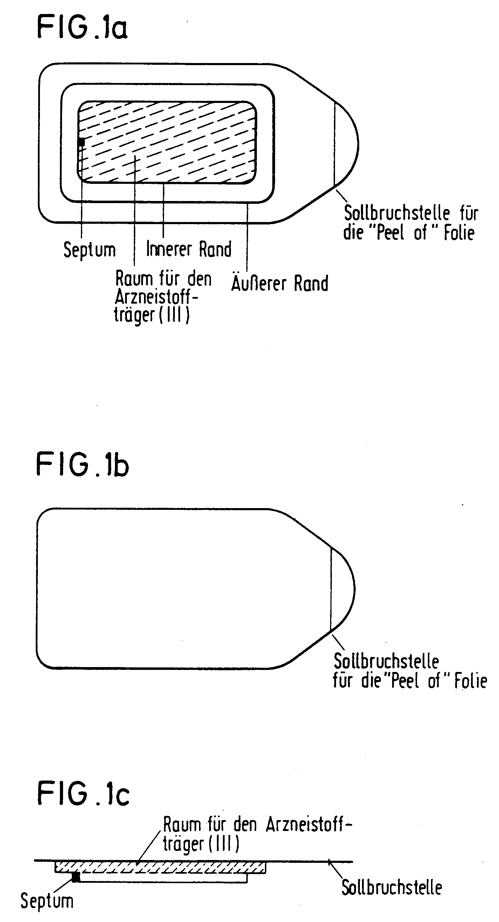

Eine erfindungsgemäße Packung wird in Fig. 1 gezeigt. Dabei stellt

Fig 1a) die Aufsicht von der Unterseite,

Fig. 1b) die Aufsicht von der Oberseite, und die Fig. 1c) eine Seitenansicht dar.

Fig. 2 zeigt eine Ausführungsform, bei der eine gestrickte Gefäßprothese in einer erfindungsgemäßen Packung vorliegt.

Der offene Behälter ist in einer besonders zweckmäßigen Ausführungsform ein flacher, tiefgezogener Behälter aus einem tiefziehfähigen Kunststoff.

Ein solcher Behälter kann leicht in Serienfertigung durch einfache Modifizierung an sich bekannter Verpackungsmaschinen hergestellt werden, z.B. durch Tiefziehen von Folien. In einem Arbeitsgang kann die Form des Behälters tiefgezogen und dabei auch schon ein Septum vorgesehen werden. Nach dem Einlegen des Arzneimittelträgers kann mit einer semipermeablen Membran, z.B. eines beschichteten Papiers, der Verschluß des Behälters erfolgen. In der Regel wird dann das Septum in die dafür vorgesehene Vorrichtung (Aus- bzw. Einsparung) aufgeklebt oder eingebracht. Man kann jedoch auch in die als Verschluß dienende semipermeable Membran das Septum einarbeiten. Das Septum kann aus Silikonkautschuk vorgefertigt sein oder wird durch Auftragen einer medizinisch annehmbaren, selbsthärtenden Silikonkautschukmasse angebracht.

Der verschlossene Behälter mit dem darin befindlichen Arzneimittelträger wird zunächst in herkömmlicher Weise sterilisiert. Der Arzneimittelwirkstoff, der nicht sterilisierbar ist, wird dann unter sterilen Kautelen durch das Septum in die Packung eingebracht. Wird nur eine einseitige Beschichtung des Arzneimittelträges mit dem Wirkstoff gewünscht, wie dies beispielsweise bei der Herstellung eines Kollagen Fibrinogen-Verbundes der Fall ist, so kann man durch die Wahl des Abstandes zwischen Träger und Behältnis die gewünschte Schichtdicke erreichen, wobei es weiterhin zweckmäßig sein känn, durch Einarbeiten eines Randes in den Behältnissen das Vordringen der mit dem nicht sterilisierbaren Wirkstoff beladenen Flüssigkeit auf eine Oberfläche des Trägers zu begrenzen.

## Beispiel 1

Das Behältnis wie in der Abb. 1 dargestellt kann durch Tiefziehen in einer entsprechenden Form erhalten werden.

Es können auch entsprechende Behältnisse aus Glas Verwendung finden. Nach Einlegung des Arzneistoffträgers werden die Behältnisse mit einer semipermeablen Membran verschlossen. Das Behältnis kann auch nach Einlegen des Arzneistoffträgers zusammengesteckt bzw. -geschraubt werden. Nach erfolgter Sterilisation erfolgt die Beschichtung bzw. Beschickung einschl. dem Abziehen von Wasser und/oder Lösungsmittel via semipermeable Membran. Das Abziehen von Lösungsmittel bzw. Wasser kann auch via Septum durch Einstecken eines Filtervorsatzes erfolgen. Der Arzneistoffträger mit dem Arzneistoff kann dann vom Anwender unter sterilen Kautelen durch Öffnen des Behältnisses entnommen werden.

In die Behältnisse werden nach dem Tiefziehen ein Träger wie Kollagen eingelegt und mit einer bakteriendichten Membran in Form von Folien, wie Tyvek oder Spezialpapier, verschlossen.

Die für die Chargen notwendige Zahl der Behältnisse mit Kollagen werden nach etablierter Technik in den dafür notwendigen Umhüllungen sterilisiert, wie z.B. mit Ethylenoxid.

Nach erfolgter Sterilisation, gegebenenfalls nach der notwendigen Ausdampfzeit bei Ethylenoxid-Sterilisation, wird Kollagen via Septum mit der Arzneistofflösung unter sterilen Kautelen beschickt oder beschichtet. Die notwendige genaue Dosierung wird durch Verwendung geeichter automatischer Dosierhilfen sichergestellt.

Werden Beschichtungen mit labilen Plasmaproteinen vorgenommen, so muß anschließend mittels der Gefriertrocknung die eingebrachte Feuchtigkeit entfernt werden, um eine Verankerung des aufgebrachten Plasmaproteins im obersten Randbereich des Arzneistoffträgers sicherzustellen. Bei diesem Verfahren wird die aufgebrachte Schicht regelrecht "auflyophilisiert".

Bei gleichmäßiger Beschickung des Arzneistoffträgers mit einem Arznmeistoff wie Gentamycin Palmitat werden entsprechend genau dosierte Mengen des Arzneistoffs in einem geeigneten Lösungsmittel wie Chloroform via Septum eingebracht, der Arzneistoffträger gleichmäßig getränkt und das Lösungsmittel in einem Vakuumtrockner mit Lösungsmittelfalle abgezogen.

Nach Trocknung bzw. Entfernung des Lösungsmittels kann unter sterilen Kautelen die Endverpackung vorgenommen werden.

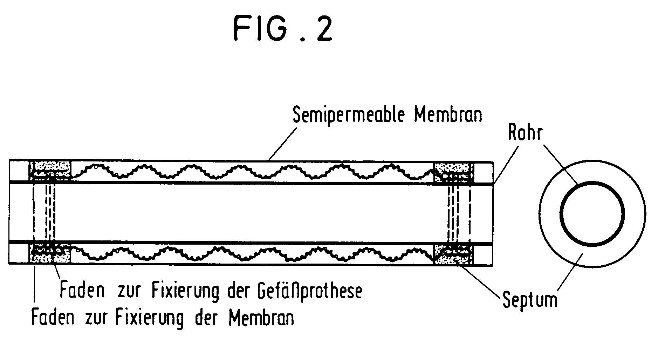

Diese Technik ist auch geeignet, um nicht resorbierbare Implantate mit Wirkstoffen zu beladen bzw. mit biologischen Substanzen zu beschichten, um diese Bioimplantate somit gewebefreundlicher zu gestalten. So kann die Biologisierung von Gefäßimplantaten nach dem in Abb. 2 aufgezeigten Schema durchgeführt werden. Mittels der aufgezeigten Behältnisse kann somit die Biologisierung von Gefäßprothesen mit Proteinen wie Kollagen durchgeführt werden einschl. der anschließend notwendigen Sterilisation. Diese Behältnisse können gleichzeitig zur Endverpackung der biologisierten Implantate dienen.

## Beispiel 2

Biologisierung einer gestrickten Gefäßprothese: Die gestrickte Prothese wird, wie in Abb. 2 schematisch gezeigt, über das Rohr gezogen und an der Einkerbung mit einem Faden fixiert. Dann wird an der bezeichneten Stelle ein Ring aus einem flexiblen Silikonmaterial über die fixierte Gefäßprothese geschoben. Ein angefeuchteter Dialyseschlauch passender Größe wird über Rohr- und Gefäßprothese gezogen und ebenfalls mit einem Faden fixiert. Die Prothese kann nun in üblicher Weise mit Gamma-Strahlung bzw. Ethylenoxidbehandlung oder Hitze sterilisiert werden.

Es besteht nun die Möglichkeit, via flexiblem Silikonring, der hier als Septum dient, unter aseptischen Bedingungen die Prothese mit einem sterilen Arzneisstoff zu beschichten. Hier findet die textile Struktur der Gefäßprothese als Arzneistoffträger Verwendung.

Es kann ferner eine 0,5%ige Kollagenlösung via Septum unter Vermeidung von Luftblasen zwischen Rohr und semipermeabler Membran eingebracht werden mit nachfolgender Gefriertrocknung und Sterilisation mittels Ethylenoxid.

Es besteht aber auch die Möglichkeit, die bereits mit Kollagen biologisierten implantierten Gefäßprothesen weiter mit einem Arzneistoff wie Gentamycin-Palmitat zu beladen.

## Patentansprüche

1. Packung mit einem darin enthaltenen, mit einem nicht sterilisierbaren Wirkstoff beladenen Wirkstoffträger, dadurch **gekennzeichnet,** daß sie durch folgende Verfahrensschritte erhältlich ist:
   a) Einbringen des Arzneimittelträgers in eine Wanne aus Kunststoff,
   b) Verschließen der Wanne mit einer semipermeablen Membran,
   c) Sterilisieren des verschlossenen Behälters,
   d) Einbringen des Wirkstoffes in gelöster oder suspendierter Form in einem Lösungsmittel durch ein Septum der Wanne oder der Membran, und
   e) Entfernen des Lösungsmittels durch die semipermeable Membran.

2. Verfahren zum Beladen von Arzneistoffträgern mit einem nicht sterilisierbaren Wirkstoff unter sterilen Kautelen, wobei der beladene Arzneimittelträger gleichzeitig steril verpackt wird, dadurch **gekennzeichnet,** daß man
   a) den Arzneimittelträger in eine Wanne aus Kunststoff einbringt,
   b) die Wanne mit einer semipermeablen Membran verschließt,
   c) den verschlossenen Behälter sterilisiert,
   d) den Wirkstoff in gelöster oder suspendierter Form in einem Lösungsmittel durch ein Septum der Wanne oder der Membran einbringt, und
   e) das Lösungsmittel durch die semipermeable Membran entfernt.

3. Verfahren gemäß Anspruch 2, dadurch **gekennzeichnet,** daß in Stufe e) das Lösungsmittel durch Gefriertrocknen oder durch Abdampfen unter vermindertem Druck, gegebenenfalls bei erhöhter Temperatur, entfernt wird.

## Claims

1. A packing containing an active ingredient carrier charged with a non-sterilizable active ingredient, characterised in that it can be produced by the following process steps:
   a) introduction of the pharmaceutical carrier into a container made of plastics material,
   b) closing of the container with a semipermeable membrane,
   c) sterilization of the closed container,
   d) introduction of the active ingredient, dissolved or suspended in a solvent, through a septum of the container or the membrane and
   e) elimination of the solvent through the semi-permeable membrane.

2. A process for charging pharmaceutical carriers with a non-sterilizable active ingredient under sterile conditions, wherein the charged pharmaceutical carrier is simultaneously packed in sterile form, characterised in that
   a) the pharmaceutical carrier is introduced into a container made of plastics material,
   b) the container is closed by a semi-permeable membrane,
   c) the closed container is sterilized,
   d) the active ingredient is introduced, dissolved or suspended in a solvent, through a septum of the container or the membrane and
   e) the solvent is eliminated through the semi-permeable membrane.

3. A process as claimed in Claim 2, characterised in that in stage e) the solvent is eliminated by freeze drying or by evaporation under reduced pressure, optionally at an increased temperature.

## Revendications

1. Emballage avec un support de substance active placé à l' intérieur, chargé d'une substance active non stérilisable, caractérisé en ce qu'il est obtenu par les étapes de procédé suivantes :

   a) introduction du support de médicament dans une auge en matière synthétique,

   b) obturation de l'auge à l'aide d'une membrane semi-l perméable,

   c) stérilisation du récipient fermé,

   d) introduction de la substance active, sous forme dissoute ou en suspension, dans un solvant, au moyen d'un septum de l'auge ou de la membrane, et

   e) élimination du solvant en passant par la membrane semi-perméable.

2.   Procédé pour remplir des supports de médicaments renfermant un produit non stérilisable dans des cautèles stériles, le support de médicament chargé étant simultanément emballé dans des conditions stériles, caractérisé en ce que l'on

   a) introduit le support de médicament dans une auge en matière synthétique,

   b) obture l'auge à l'aide d'une membrane semi-perméable,

   c) stérilise le récipient fermé,

   d) introduit la substance active, sous forme dissoute ou en suspension, dans un savant, au moyen d'un septum de l'auge ou de la membrane, et

   e) élimine le solvant en passant par la membrane semi-perméable.

3.   Procédé selon la revendication 2, caractérisé en ce qu'à l'étape e), le solvant est éliminé par séchage par congélation ou par vaporisation sous une pression réduite, le cas échéant à température élevée.

## FIG.1a

Septum | Innerer Rand
Raum für den Arzneistoffträger (III) | Äußerer Rand
Sollbruchstelle für die "Peel of" Folie

## FIG.1b

Sollbruchstelle für die"Peel of" Folie

## FIG.1c

Raum für den Arzneistoffträger (III)
Septum
Sollbruchstelle

# FIG . 2

Semipermeable Membran

Rohr

Faden zur Fixierung der Gefäßprothese
Faden zur Fixierung der Membran

Septum